# EUROPEAN PATENT SPECIFICATION

(11) **EP 1 748 296 B1**
(45) Date of publication and mention of the grant of the patent: **24.02.2010**
(21) Application number: 06425525.0
(22) Date of filing: 25.07.2006
(51) Int. Cl.: G01N 33/546, G01N 33/50

(54) **Method for detecting GAD65 autoreactive T cells**
Verfahren für den Nachweis von GAD65 selbstreaktiven T-Zellen
Procédé de détection des cellules T autoréactives contre GAD65

(30) Priority: 26.07.2005 IT RM20050398
(43) Date of publication of application: 31.01.2007
(73) Proprietor: Fierabracci, Alessandra, 00167 Roma (IT); Bottazzo, Gian Franco, 00186 Roma (IT)
(72) Inventor: Fierabracci, Alessandra, 00167 Roma (IT)
(74) Representative: Capasso, Olga

(56) References cited:
- WO-A-02/080963
- ANONYMOUS: "Attività Scientifica 2004" INTERNET ARTICLE, [Online] 2004, page 3,9,283,284, XP002400134 & XI Incontro di Informazione Scientifica, Ospedale Bambin Gesu, Rome, Italy; July 14-15, 2004 Retrieved from the Internet: URL:http://www.ospedalebambinogesu.it/item /2625/as2004_e.pdf> [retrieved on 2006-09-21]
- PANINA-BORDIGNON PAOLA ET AL: "Cytotoxic T cells specific for glutamic acid decarboxylase in autoimmune diabetes" JOURNAL OF EXPERIMENTAL MEDICINE, vol. 181, no. 5, 1995, pages 1923-1927, XP002400135 ISSN: 0022-1007
- KITA H ET AL: "Application of tetramer technology in studies on autoimmune diseases" AUTOIMMUNITY REVIEWS, vol. 2, no. 1, January 2003 (2003-01), pages 43-49, XP002375628 ISSN: 1568-9972
- NEPOM GERALD T: "MHC multimers: Expanding the clinical toolkit." CLINICAL IMMUNOLOGY, vol. 106, no. 1, January 2003 (2003-01), pages 1-4, XP002400136 ISSN: 1521-6616
- OGG GRAHAM S ET AL: "High frequency of skin-homing melanocyte-specific cytotoxic T lymphocytes in autoimmune vitiligo" JOURNAL OF EXPERIMENTAL MEDICINE, vol. 188, no. 6, 21 September 1998 (1998-09-21), pages 1203-1208, XP002400138 ISSN: 0022-1007
- BARBER LINDA D ET AL: "Detection of vimentin-specific autoreactive CD8+ T cells in cardiac transplant patients" TRANSPLANTATION, vol. 77, no. 10, 27 May 2004 (2004-05-27), pages 1604-1609, XP002400139 ISSN: 0041-1337
- EISENBARTH GEORGE S ET AL: "Enumerating autoreactive T cells in peripheral blood: A big step in diabetes prediction." JOURNAL OF CLINICAL INVESTIGATION, vol. 111, no. 2, January 2003 (2003-01), pages 179-181, XP002400137 ISSN: 0021-9738
- FIERABRACCI A ET AL: "Detection of GAD(65)-specific T cells by class I tetramers in newly diagnosed type 1 diabetes (TID) patients" DIABETOLOGIA, vol. 48, no. Suppl. 1, 2005, pages A98-A99, XP002400140 & 41ST ANNUAL MEETING OF THE EUROPEAN-ASSOCIATION-FOR-THE-STUDY-OF-DIAB ETES; ATHENS, GREECE; SEPTEMBER 10 -15, 2005 ISSN: 0012-186X

## Description

### TECHNICAL FIELD OF INVENTION

The present invention concerns a method for detecting in a biological sample T cells reacting with the autoantigen glutamic acid decarboxylase, isoform 65 (GAD65) by using specific ligands, i.e. tetramers of the major histocompatibility complex HLA class I. The method has certain advantages and allows the early diagnosis of insulin-dependent diabetes mellitus (Type 1 diabetes, T1D) and the monitoring of the disease outcome in patients receiving this early diagnosis. The method is particularly advantageous for patients with T1D and for individuals at high risk (islet related-autoantibody positive) to develop T1D (secondary prevention).

### STATE OF THE TECHNOLOGY

Type 1 Diabetes (T1D) is an autoimmune disease in which the insulin-producing pancreatic beta cells are destroyed in genetically predisposed individuals. The pre-clinical onset of the disease is characterized by the presence of circulating autoantibodies directed against pancreatic islet specific autoantigens. The contribution of autoantibodies to the disease pathogenesis is still controversial, whereas it is generally accepted that autoreactive T lymphocytes, which have escaped the thymic selection process, are indeed responsible for beta cell autoimmune destruction. However, surprisingly, several attempts to standardize a reliable and reproducible assay for detecting and/or measuring autoreactive T cells to islet autoantigens in the circulation have been unsuccessful (Peakman 2001; Fierabracci, Bottazzo 2002). Several explanations have been proposed for the encountered difficulties, for example, the low precursor frequency of circulating autoreactive T cells and the fact that they should be preferentially recruited in the lesion in the organ (unfortunately inaccessible at the time of diagnosis), or the molecular features of the autoantigenic proteins employed in the different techniques.

Therefore, the aim of the present invention is to provide a reliable and reproducible technology for the detection of circulating GAD65 autoreactive cytotoxic T lymphocytes in newly diagnosed T1D patients. The proposed technology relies on the use of HLA class I tetrameric complexes.

Until now, only a few papers have described the successful use of tetramers for phenotyping of T cells in models of human autoimmune disease. The first demonstration (Kotzin, 2000) reports the potential use of biotinylated HLA class II DR4 tetramers constructed using epitopes of synovial autoantigens of collagen II and cartilage antigen HCgp39. The study aimed to define the specific determinants against which the major oligoclonal response of CD4+ T cells is preferentially directed in rheumatoid arthritis. Very low percentages of reactive T cells were detected by flow cytometric analysis of the lymphocytic population infiltrating synovial tissue. In the experimental procedure, the cells were not subjected to preliminary antigen-specific expansion. Subsequently (Reijonen 2002; reviewed in Reijonen 2003), soluble HLA-DR 401 or HLA-DR 404 class II tetramers constructed with a GAD65 immunodominant epitope were used. These complexes were employed to detect CD4+ T lymphocytes of T1D patients (analysed between 1 to 6 months from diagnosis) and of subjects at high risk to develop T1D. Because the aim was to detect autoreactive T cells, presumably circulating at very low concentrations, a preliminary expansion of lymphocytes in culture, by incubation with the GAD65 peptide, was introduced. After 6 to 10 days, lymphocytes were activated by culturing them in wells of special plates, whose internal surface had adsorbed HLA class II monomers expressing the same peptide. After 48 hours, a low dose of IL-2 was added. On the third day of activation, cells were stained with phycoerythrin conjugated tetrameric complexes and analysed by flow cytometry. CD4+/CD25+ activated T cells were detected in all the T1D patients and high-risk individuals, but not in the healthy controls. There are two main limitations of the study: i) the analysis was carried out with a too-small number of patients and control subjects; and ii) a non-natural GAD65 epitope (555-567) was used, whose affinity binding to HLA class II molecules was increased as the result of the substitution of one amino acid in the original sequence (F→I in position 557).

In a subsequent publication (Forneau, 2004), evidence of specific staining of CD4+ T cells by means of GAD65 272-285 and IA2 718-729 tetramers, constructed with 'natural' epitopes, was reported. However, these reagents were not used in an extended analysis of T1D patients and healthy controls.

The first demonstration of the successful detection of CD8+ cytotoxic T lymphocytes by means of HLA class I tetramers, constructed with an epitope of the MelanA autoantigen, was reported in patients with vitiligo (Ogg, 1998). One limitation of the study is that, even in this case, the epitope 26-35, selected from the natural protein sequence, has been modified by substitution of alanine in position 2 with leucine, in order to increase the affinity binding to the HLA-A*0201 haplotype. CD8+ lymphocytes were detected in a direct assay ex-vivo by flow cytometric analysis. Alternatively, lymphocytes of patients and controls were expanded in the presence of IL-7 and peptide and the clones used in cytotoxicity tests. This method of direct assay ex-vivo was not successful when using HLA class I tetramers constructed with an epitope of the tyrosinase autoantigen.

A subsequent study reported the detection of autoantigen vimentin-specific autoreactive CD8+ T cells in cardiac transplant patients using HLA class I tetramers (Barber, 2004), supporting the evidence for a secondary T cell-mediated organ-specific autoimmune response. Tetramers were constructed with a combination of HLA-A*0201 biotinylated protein and phycoerythrin-conjugated streptavidin. Analysis of the culture was performed after 21-28 days of antigen-specific stimulation.

The instant invention renders possible to label CD8+ cytotoxic T cells with HLA class I tetramers constructed with a natural epitope of GAD65. One publication has reported the presence of GAD65-specific cytotoxic T cells in autoimmune diabetes (Panina-Bordignon, 1995), detected by a cytotoxicity test performed with a GAD65 decamer (GAD 114-123), that differs from the object of the present invention (GAD 114-122) by the presence of a terminal valine (vide infra). In the same study, peripheral blood lymphocytes, after Ficoll gradient separation, were cultured with their own dendritic cells (adherent to the plate after two hours of incubation), pre-pulsed overnight with the GAD65 peptide 114-123. The peptide used in that study derived from an affinity selection, in which the HLA-A*0201 molecule was exposed by T2 cells. During the generation of the GAD65 reactive lines, at day 5, lymphocytes were supplemented with IL-2 (10 IU/ml) and the cytotoxicity test performed at day 8.

The patent application WO 01/13934 describes GAD65 peptide analogues. These peptides antagonize the T cell-mediated GAD65 activation, and can be used for the treatment and prevention of T1D (Gebe 2004). The sequence of the described peptides is different from that of the peptide of the present invention. The patent application WO 02/080963 describes the use of tetrameric complexes to identify immunostimulatory epitopes of relevant autoantigens, such as GAD65. However, these complexes are HLA class II tetramers, whereas the tetramers of the present invention are HLA class I.

The summary of scientific activities of the Pediatric Hospital Bambino Gesu, 2004, p. 283-284 discloses a method for detecting GAD65 specific T cells in a biological sample, however the document does not describe all steps of the present method, in particular it does not disclose a double GAD-cytokine stimulation.

The present invention describes the detection of autoreactive CD8+ cytotoxic T cells in

T1D using HLA class I tetramers constructed with a natural nonapeptide (VMNILLQYV, SEQ ID No. 1) obtained from the sequence of the GAD65 autoantigen and selected according to database prediction algorithms of affinity binding to the HLA-A *0201 allele, which is highly superior to that of the GAD decamer 114-123 (VMNILLQYVV, SEQ ID No. 2) peptide. The technology used has been validated in a statistically significant number of patient samples, affected by T1D (recruited within one week of diagnosis) and a comparable number of healthy controls.

Due to the rarity of the GAD65 autoreactive T cells that the technology aims to detect in the peripheral blood, a short period of antigen-specific stimulation has been introduced as a preliminary step before tetramer staining and subsequent flow cytometric analysis. The absolute specificity of the tetramer-T lymphocyte binding is assured by the introduction of an intermediate washing of the stimulated lymphocyte population, followed by an interval of stimulation with IL-2 only and in the absence of the GAD65 peptide. This is therefore the first example of a validated technology, using HLA class I tetramers constructed with a natural epitope, in a model of primary human autoimmune disease, such as T1D.

### DESCRIPTION OF THE INVENTION

Therefore the object of the present invention is an *in vitro* method for detecting glutamic acid decarboxylase, isoform 65 (GAD65) specific T cells in a biological sample that consists of the following steps:
a) isolating mononuclear cells (PBMCs) from peripheral blood obtained from a subject;
b) stimulating said isolated PBMCs with an appropriate amount of the GAD65 peptide having the sequence VMNILLQYV (SEQ ID NO .1) added to the culture medium, in order to obtain autoantigen-specific stimulated PBMCs;
c) washing at least once said autoantigen-specific stimulated PBMCs;
d) stimulating said autoantigen-specific stimulated PBMCs with an appropriate amount of at least one cytokine to obtain fully stimulated PBMCs;
e) incubating in appropriate conditions said fully stimulated PBMCs with an appropriate amount of multimeric HLA class I labelled complexes, constructed with a peptide derived from GAD65 having the sequence VMNILLQYV (SEQ ID No. 1) and wherein the multimeric complexes are tetrameric or pentameric.
f) identifying and quantifying the percentage of T cells that are bound to said complexes and comparing said percentage to appropriate control values. By "peptide derived from GAD65", we mean a peptide of amino acid sequence enclosed within the GAD65 sequence, as described by Strausberg et al (2002) and P48320, Database National Center for Biotechnology Information (NCBI).

The cytokine is preferably IL-2 and the complexes are preferably labelled with phycoerythrin.

It is another object of the invention an *in vitro* method for the prediction of Type 1 diabetes (T1D) onset, and/or to follow up the effects of therapeutics and in particular of specific immunotherapies in the pre-clinical period of the disease, essentially consisting of the steps of the method according to the previous claims.

A further object of the present invention is a 'kit' able to work the method of the invention including:
- a peptide derived from GAD65 having the sequence VMNILLQYV (SEQ ID No. 1);
- at least one cytokine;
- a multimeric labelled complex composed of HLA class I and the peptide derived from GAD65, wherein the multimeric complex is tetrameric or pentameric;
- a monoclonal antibody labelled with a first fluorochrome, directed against the human CD8 molecule; and
- a monoclonal antibody labelled with a second fluorochrome, directed against the human CD3 molecule.

Preferably, the cytokine is IL-2 preferably the complex is labelled with phycoerythrin. In particular, Cy5 is the first fluorochrome labelling the monoclonal antibody directed against the human CD8 molecule and allophycocyanin (APC) is the second fluorochrome labelling the monoclonal antibody directed against the human CD3 molecule.

The present invention will be described in the following non limitative examples, especially referred to the following Figures.

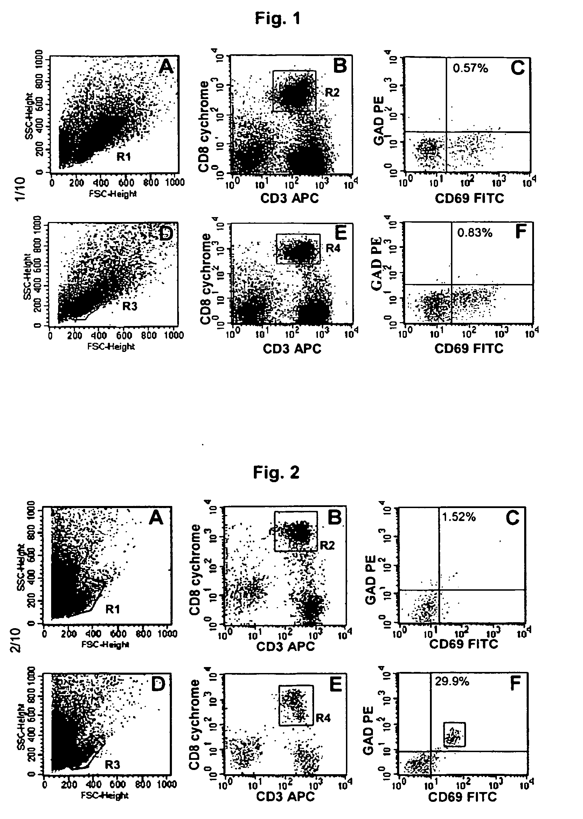

Figure 1 Dot plots of cytometric analysis (FSC-H and SSC-H) of PBMCs of a representative healthy control donor at day 6 from the beginning of the culture, (A-C): cells stimulated with IL-2, (D-F): cells stimulated with the GAD65 peptide. Gates R1 and R3 represent lymphocyte populations treated with IL- 2 or GAD65 peptide. B and E: dot plots of fluorescence intensities of CD3 APC and CD8 Cychrome of lymphocyte populations of gate R1 of panel A and R3 of panel D after (B) IL-2 treatment or (E) GAD65 peptide treatment. R2 and R4 circumscribe CD3⁺/CD8⁺ positive cells in the lymphocyte populations. C and F: dot plots of fluorescence intensities of CD69 FITC and GAD65 tetramer PE of gates R2 and R4 after (C) IL-2 treatment or (F) GAD65 peptide treatment.

Figure 2 Dot plots of cytometric analysis (FSC-H and SSC-H) of PBMCs of a representative T1D patient at day 6 from the beginning of the culture, (A-C): cells stimulated with IL-2, (D-F): cells stimulated with GAD65 peptide. Gates R1 and R3 represent lymphocyte populations treated with IL-2 or GAD65 peptide. B and E: dot plots of fluorescence intensities of CD3 APC and CD8 Cychrome of lymphocyte populations corresponding to R1 of panel A and R3 of panel D after (B): IL-2 treatment or (E) GAD65 peptide treatment. R2 and R4 circumscribe CD3⁺/CD8⁺ positive cells in the lymphocyte populations. C and F: dot plots of fluorescence intensities of CD69 FITC and GAD tetramer PE of gates R2 and R4 after (C): IL-2 treatment or (F): GAD65 peptide treatment.

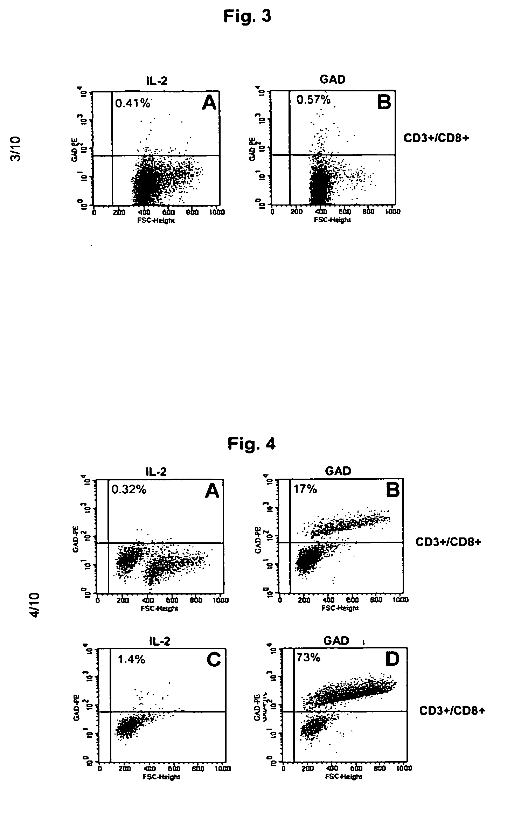

Figure 3 Dot plots of flow cytometric analysis of PBMCs of a representative healthy control at day 6 from the beginning of culture (number 22 in Table 3). (A): cells stimulated with IL-2, (B): cells stimulated with GAD65 peptide. The percentage of CD3+/CD8+/GAD65 reactive T lymphocytes is shown.

Figure 4 Dot plots of cytometric analysis of PBMCs of a representative T1D patient (A, B: patient number 2 of Table 4, C, D: patient number 1 of Table 4) at day 6 from the beginning of the culture, (A,C): cells stimulated with IL-2 , (B,D): cells stimulated with the GAD65 peptide. The percentage of CD3+/CD8+/GAD65 reactive T lymphocytes is shown.

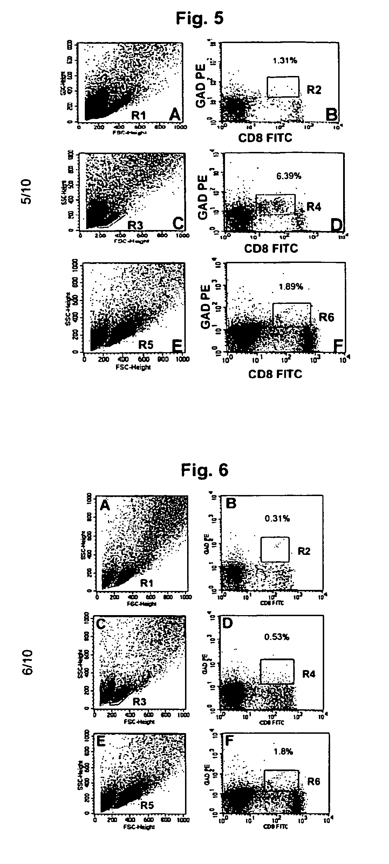

Figure 5 Dot plots of cytometric analysis (FSC-H and SSC-H) of the lymphocyte population of a representative T1D patient at day 6 from the beginning of culture, (A, B): cells stimulated with Flu peptide, (C,D): cells stimulated with the GAD65 peptide; (E-F): cells in basal conditions of IL-2 stimulation. R2, R4 and R6 circumscribe the CD8+/GAD65 reactive T lymphocytes. The percentage of CD8+/GAD65 reactive T cells in the lymphocyte populations is shown.

Figure 6 Dot plots of flow cytometric analysis (FSC-H and SSC-H) of the lymphocyte population of a representative healthy control individual at day 6 from the beginning of culture; (A, B): cells stimulated with Flu peptide; (C,D): cells stimulated with the GAD65 peptide; (E-F): cells in basal conditions of IL-2 stimulation. R2, R4 and R6 circumscribe the population of CD8+/GAD65 reactive T lymphocytes. The percentage of CD8+/GAD65 reactive T cells in the lymphocyte populations is shown.

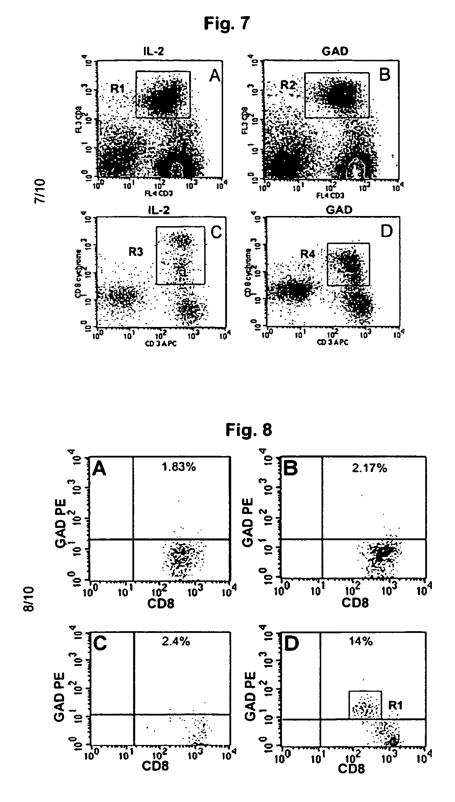

Figure 7 Dot plots of flow cytometric analysis of the total CD3+/CD8+ lymphocyte population, (A,B) of a healthy control individual and (C,D) of a T1D patient at day 6 from the beginning of culture, (A,C) cells IL-2 stimulated, (B-D) cells stimulated with GAD65 peptide. R2 and R4 circumscribe the CD3+/CD8+ cells. R4 shows a peculiar pattern of 'low CD8' expression.

Figure 8 Representative dot plots of fluorescence intensities of CD3+/CD8+/GAD65 reactive T lymphocytes (A,B) of a healthy control individual and (C,D) of a T1D patient at day 4 from the beginning of culture, (A,C) cells stimulated with IL-2, (B,D) cells stimulated with the GAD65 peptide. R1 indicates the CD8+/GAD65 reactive T lymphocytes of the T1D patient. The percentage of CD8+/GAD65 reactive T cells in the lymphocyte populations is shown.

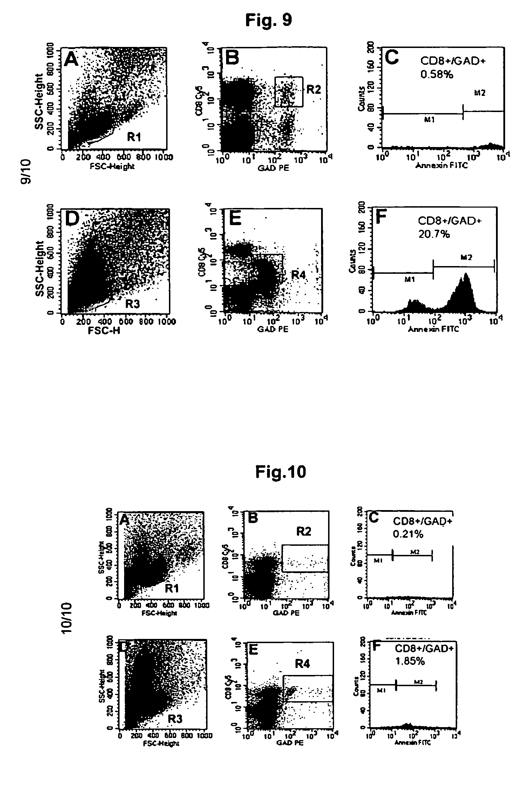

Figure 9 Representative dot plots of flow cytometric analysis (FSC-H and SSC-H) of the lymphocyte population (A-C) of a healthy control individual and (D-F) of a T1D patient at day 4 from the beginning of culture, after stimulation with GAD65 peptide. R1 and R3 indicate the lymphocyte populations. B and E represent dot plots of fluorescence intensities of CD8 Cy5 and GAD tetramer PE (B) of a healthy control individual and (E) a T1D patient. R2 and R4 circumscribe the CD8+/GAD65 reactive lymphocyte population. C and

F represent histogram plots of fluorescence intensities of annexin V positive cells in R2 and R4, respectively. The percentage of CD8+/GAD reactive/annexin V + T cells in the lymphocyte populations is shown.

Figure 10 Representative dot plots of flow cytometric analysis (FSC-H and SSC-H) of the lymphocyte population (A-C) of a healthy control individual and (D-F) of the T1D patient at day 6 from the beginning of culture, after stimulation with the GAD65 peptide. R1 and R3 represent the lymphocyte populations. B and E represent dot plots of fluorescence intensities of CD8 Cy5 and GAD tetramer PE (B) of a healthy control individual and (E) of a T1D patient. R2 and R4 circumscribe the CD8+/GAD reactive population. C and F represent histogram plots of fluorescence intensities of annexin V positive cells in R2 and R4, respectively. The percentage of CD8+/ GAD reactive/ annexin V+ T cells in the lymphocyte populations is shown.

### MATERIALS AND METHODS

### Selection of the GAD65 peptide

In humans, 3 different molecules of the major histocompatibility class I complex have been characterized: A, B, C. They are highly polymorphic (up to 800 different alleles) and are coded by alleles derived from paternal and maternal chromosomes; therefore each individual is able to express up to 6 different HLA class I molecules. The function of the HLA class I molecule is strictly dependent on its structure. The site of peptide binding presents 6 pockets (A-F), where residues of peptide anchoring (preferably nonapeptides) may be located. The allelic variants select the repertoire of peptides that may be located in each site of HLA class I. The HLA-A2 allele is represented in approximately half the general population and the subtype *0201 is expressed in more than than 90% of HLA-A2 positive individuals. Because its frequency is so high, the HLA-A*0201 allele has been considered in the present invention. The GAD65 peptide was selected by Database search for its high affinity binding to the pocket HLA-A*0201 (http://www-bimas.nih.gov/cgi-bin/molbio/ken parker comboform). The peptide of sequence VMNILLQYV (named GAD65 peptide, SEQ ID No. 1) was selected in position 114-122 of the GAD65 sequence.

### Subjects

Blood samples were obtained at disease onset from pediatric patients with T1D (n=22, aged 3 months to 15 years) from the Endocrinology Unit of Bambino Gesù Children's Hospital in Rome. All patients were positive for autoantibodies directed against one or more diabetes-related autoantigenic specificities: ICA, anti-GAD65, anti-IA-2 and anti-insulin. One third of patients were negative for GAD autoantibodies. Only subjects found positive on molecular analysis for the haplotype HLA-A2 were included in the study. The control population comprised 22 healthy HLA-A2 positive subjects, asymptomatic, with no family history of autoimmune disease, and without circulating T1D-related autoantibody specificities.

### Isolation and stimulation of peripheral blood mononuclear cells (PBMCs)

PBMC were separated from 5-10 ml heparinized peripheral blood by Ficoll gradient centrifugation (Histopaque, Sigma-Aldrich, St. Luis, MO, USA). The cells were resuspended in RPMI-1640 (GIBCO/BRL, Invitrogen, Gaithersburg CA, USA), supplemented with 2 mmol/l L-glutamine, 100 µg/ml penicillin/streptomycin and 10% vol/vol fetal bovine serum (FBS, Hyclone, South Logan, UT, USA) at a density of 1X10⁶/ml; cells were cultured in 24-well plates (Falcon, Labware BD Biosciences, Oxnard, CA, USA) in the presence of the GAD65 peptide added to the culture medium at a concentration ranging from 10 to 30 µg/ml or in the presence of IL-2 (at a concentration of 25 IU/ml). At day 4 from the beginning of the culture, both GAD65 peptide stimulated PBMCs and IL-2 treated PBMCs were washed in calcium-magnesium free Dulbecco's phosphate buffered saline 1X (Euroclone, Wetherby, West York, UK), by centrifugation at 1500 rpm for 5 min at room temperature. This washing step was introduced in the method to remove the GAD65 peptide and therefore reduce the risk of aspecificities in the subsequent procedure of tetramer binding. Both the cells that had been previously stimulated with the GAD65 peptide and the IL-2 stimulated cells were subsequently cultured in the same medium [RPMI-1640, supplemented with L-glutamine (2mmol/l), penicillin/streptomycin (100 µg/ml) and fetal bovine serum (10% vol/vol)] in the presence of IL-2 (25 IU/ml) for an additional 2 days. In further experiments (Fig 8), both GAD65 peptide stimulated PBMCs and IL-2 treated PBMCs were washed in PBS at day 2, instead of day 4, and then both cultured in the presence of IL-2 for an additional two days as previously indicated. Cells were then harvested and processed for tetramer staining and flow cytometric analysis.

Mononuclear cells of the same samples obtained from T1D patients and controls were cultured in the presence of the control peptide Flu GILGFVFTL (SEQ ID No. 3)of the influenza virus, known to have high affinity binding to HLA-A*0201. In parallel, the culture of lymphocytes of the same samples was set up in the presence of the GAD65 peptide or in basal conditions of IL-2 stimulation. Flu and GAD65 peptides were used at the same concentration (10-30 µg/ml). At day 4 from the beginning of the experiment, cells treated in the different conditions were washed and then incubated for an additional 2 days with IL-2 before being stained with the same tetramer constructed with the GAD65 peptide and analysed by flow cytometry.

### HLA-peptide class I tetrameric complexes and flow cytometric analysis

T lymphocytes, by means of T cell receptor (TCR), recognize the HLA peptide complex on the surface of cells that present antigen. Contrary to antibodies, TCRs generally have a much lower affinity binding to their own antigen (i.e., HLA-peptide complex). Therefore, in order to increase the affinity binding, HLA-peptide complexes are engineered to form a multimer composed of 4 or 5 units (tetramer or pentamer, respectively). Complexes are labelled with different fluorochromes, e.g., phycoerythrin, which is the most commonly used because it ensures a very strong signal. As a consequence, T cells that bind the complex can be detected on flow cytometric analysis. Complexes are constructed with a combination of the heavy chain of purified HLA, β-2 microglobulin and the peptide.

Complexes of HLA class I-peptide- tetramers are used to label CD8+ cytotoxic T lymphocytes. Complexes constructed with HLA class II-peptide tetramers are used to label CD4+ T lymphocytes.

HLA class I-peptide tetrameric or pentameric complexes (Fig 5 and 6) were purchased from ProImmune Limited (Oxford, UK) and are constituted, as described above, of 4 or 5 complexes of the HLA class I molecule-peptide. These were synthesized using the GAD65 peptide VMNILLQYV (SEQ ID No. 1) that has high affinity binding to HLA-A*0201 and labelled with phycoerythrin. To verify the specificity of the binding of the GAD65 tetramer to the GAD65 peptide stimulated lymphocytes, control tetrameric complexes were synthesized using the Flu peptide GILGFVFTL (SEQ ID No. 3)that has high affinity binding to the HLA-A*0201.

On day 4 or day 6 from the beginning of culture, 1X10⁶ cells were allocated for staining. They were washed by centrifugation at 1500 rpm for 5 min at room temperature in wash buffer [0.1% sodium azide, 2% FBS in PBS] then resuspended in the residual volume (approximately 50 µl). 1 µl of phycoerythrin (PE) labelled GAD65 tetramer was added and incubated in ice for 30⁺ min in the dark. Cells were then washed in wash buffer, as described above, 1 µl of MoAb anti-human CD8 (FITC labelled, purchased from Becton Dickinson, Pharmingen, San Diego, CA, USA) and 5 µl of MoAb anti-human CD3 [allophycocyanin (APC) labelled, purchased from Becton Dickinson] were then added per staining condition. Cells were incubated in ice for at least 30 min in the dark. Cells were then washed twice in phosphate buffer and their staining achieved using a Beckton Dickinson FACSCalibur flow cytometer and analysed with the CellQuest software program. T cell activation was evaluated by staining cells with PE-labelled GAD tetramer in different multiple staining experiments. These were done by staining cells with the phycoerythrin-labelled GAD65 tetramer, MoAb anti-human CD69 (FITC-labelled, 1:10 dilution, purchased from Becton Dickinson) and a biotinylated anti-human CD8 Moab (1:10 dilution, Becton Dickinson); the reactivity of the latter MoAb was detected using streptavidin-Cychrome (Cy5) conjugate (dilution 1:10, purchased from Southern Biotechnology, Birmingham, AL, USA).

### Evaluation of early-late apoptosis in peptide stimulated PBMC

At day 4 and day 6 from the beginning of the culture, 1x10⁶ cells stimulated with the GAD65 peptide according to the above mentioned protocols were washed in the same washing phosphate buffer as described above and resuspended in the residual volume of approximately 50 µl. 1 µl PE- labelled GAD65 tetramer was used per staining procedure. Cells were then incubated with biotinylated anti-human CD8 MoAb (1:10 dilution, Becton Dickinson) in ice, for at least 30 min, in the dark. They were then washed with washing buffer by centrifugation at 1500 rpm for 5 min at room temperature. 5 µl of streptavidin-(Cy5) conjugate was then added for 30 min at room temperature, in the dark. Cells were then washed in ice cold PBS (2% FBS) by centrifugation at 1500 rpm for 5 min at room temperature. Cell pellet was then suspended in 500 µl of phosphate buffer. 10 µl of cold media binding reagent (Calbiochem, San Diego, CA, USA) and 1.25µl of Annexin V-FITC conjugate (Calbiochem) were added according to manufacturer's instructions. After incubation for 15 min at room temperature, in the dark, cells were washed by centrifugation and gently suspended in 500 µl of binding buffer [from a stock solution composed of 50 mM Hepes, ph7.4, 750mM NaCl, 12.5 mM CaCl₂ , 5mM MgCl₂, 20% bovine serum albumin (BSA)] to which 10 µl of a solution of 30 µg/ml propidium iodide in phosphate buffer were added. Staining was then immediately achieved and analysed by flow cytometry. Propidium iodide was used to distinguish between viable, early apoptotic, late apoptotic and necrotic cells. Cells were evaluated as necrotic when stained with propidium iodide but negative for Annexin V, cells were evaluated as late apoptotic when propidium iodide positive and binding Annexin V, early apoptotic when propidium iodide negative and Annexin V positive and viable when negative for both stainings.

### RESULTS

### Selection of the GAD65 peptide

From Database search (performed with nonamers because class I binds residues of 8-10 aminoacids and preferably nonapeptides), the authors selected peptide GAD65 114-122 for its high score of affinity binding to HLA-A*0201 (1080.24 score) as shown in Table 1.

**Table 1. Database search of nonamers of the GAD65 protein sequence that have affinity binding to HLA A *0201. Peptide GAD65 114-122 has high affinity binding. Peptide GAD 65 115-123 has a very low affinity binding.**

| Rank | Start position | Subsequence | Score* | |
|---|---|---|---|---|
| 1 | 141 | LLQEYNWEL | 1116.719 | (SEQ ID No. 4) |
| 2 | 114 | VMNILLQYV | 1080.239 | (SEQ ID No. 1 ) |
| 3 | 573 | FLIEEIERL | 454.063 | (SEQ ID No. 5) |
| 4 | 110 | FLQDVMNIL | 379.930 | (SEQ ID No. 6) |
| 5 | 159 | ILMHCQTTL | 134.369 | (SEQ ID No. 7) |
| 6 | 456 | WLMWRAKGT | 126.833 | (SEQ ID No. 8) |
| 7 | 269 | ALPRLLAFT | 94.268 | (SEQ ID No. 9) |
| 8 | 181 | QLSTGLDMV | 78.385 | (SEQ ID No. 10) |
| 9 | 250 | AMMIARFKM | 76.998 | (SEQ ID No. 11 ) |
| 10 | 536 | RMMEYGTTM | 54.430 | (SEQ ID No. 12) |
| 11 | 213 | FVLLEYVTL | 47.721 | (SEQ ID No. 13) |
| 12 | 396 | KMMGVPLQC | 46.465 | (SEQ ID No. 14) |
| | | | | |
| 109 | 257 | RMFPEVKEK | 0.321 | (SEQ ID No. 15) |
| 110 | 115 | MNILLQYVV | 0.316 | (SEQ ID No. 16) |
| 111 | 118 | LLQYVVKSF | 0.291 | (SEQ ID No. 17) |
| 112 | 158 | EILTHCQTT | 0.284 | (SEQ ID No. 18) |
| 113 | 272 | RLIAFTSEH | 0.283 | (SEQ ID No. 19) |
| 114 | 66 | AATGKVACT | 0.238 | (SEQ ID No. 20) |
| 115 | 431 | KHYDLSYDT | 0.238 | (SEQ ID No. 21) |

| | | | | |
|---|---|---|---|---|
| *estimate of half time of disassociation of a molecule containing this subsequence | | | | |

The authors chose the peptide in second position for its affinity binding and not the first (GAD65 141-149) because peptide GAD65 114-122 has the same sequence as decamer GAD65 114-123, but without the terminal valine, and its biological significance has been demonstrated (Panina-Bordignon, 1995). However, as shown in Table 2 this decamer has a very low affinity binding with the HLA-A*0201 molecule (35.01 score), indicating that the subtraction of the terminal valine plays a key role in the presentation of the motif.

**Table 2 . Database search of decamers of the GAD65 protein sequence binding to HLA-A*0201. Peptide GAD65 114-123 has high affinity binding.**

| Rank | Start position | Subsequence | Score* | |
|---|---|---|---|---|
| 1 | 536 | RMMEYGTTMV | 762.019 | (SEQ ID No.22) |
| 2 | 203 | NMFTYEIAPV | 427.474 | (SEQ ID No. 23) |
| 3 | 110 | FLQDVMNILL | 402.895 | (SEQ ID No. 24) |
| 4 | 205 | FTYEIAPVFV | 320.650 | (SEQ ID No. 25) |
| 5 | 372 | LMSRKHKWKL | 196.712 | (SEQ ID No. 26) |
| 6 | 443 | ALQCGRHVDV | 69.552 | (SEQ ID No. 27) |
| 7 | 91 | FLHATDLLPA | 52.561 | (SEQ ID No. 28) |
| 8 | 140 | ELLQEYNWEL | 44.460 | (SEQ ID No. 29) |
| 9 | 114 | VMNILLQYVV | 35.012 | (SEQ ID No. 2) |
| 10 | 276 | FTSEHSHFSL | 32.455 | (SEQ ID No. 30) |
| 11 | 180 | NQLSTGLDMV | 29.405 | (SEQ ID No. 31) |
| 12 | 141 | LLQEYNWELA | 28.844 | (SEQ ID No. 32) |

| | | | | |
|---|---|---|---|---|
| *estimate of half time of disassociation of a molecule containing this subsequence | | | | |

The authors also observed the importance of this aminoacid residue considering the fact that the nonamer obtained from the elimination of the initial valine has a very low affinity binding (number 110, MNILLQYVV, (SEQ ID No. 16) score 0.316, Table 1)

### GAD65 reactive T cells in PBMC of healthy individuals and newly diagnosed T1D patients

### a) Healthy individuals

A low percentage of CD3+/CD8+/GAD65 reactive T cells were detected in PBMC of 22/22 normal individuals at day 6 from the beginning of culture, both after IL-2 stimulation (average 0.4%) and after GAD65 peptide stimulation (average 0.9%) (Table 3, Fig 1, Fig 3).

**Table 3: Percentage of CD3/CD8 positive GAD65 reactive T cells detected in PBMC of healthy control individuals in both basal conditions of IL-2 stimulation and under GAD65 peptide stimulation and analysed at day 6 from the beginning of culture.**

| Normal controls | IL2-treated PBMC | GAD65- treated PBMC |
|---|---|---|
| | %CD3+/CD8+/GAD65 reactive T cells | %CD3+/CD8+/GAD65 reactive T cells |
| 1 | 0.1 | 0.37 |
| 2 | 0.79 | 0.42 |
| 3 * | 0.16 | 2 |
| 4 | 0.01 | 0.09 |
| 5 | 0.45 | 0.97 |
| 6 | 0 | 0.6 |
| 7 * | 0.2 | 8 |
| 8 | 1.07 | 1.21 |
| 9 | 0.11 | 0.25 |
| 10 | 0.16 | 0.9 |
| 11 | 1.28 | 1.03 |
| 12 | 0.57 | 0.49 |
| 13 | 0.17 | 0.23 |
| 14 | 0.11 | 0.03 |
| 15 | 0.27 | 0.21 |
| 16 | 0.23 | 0.11 |
| 17 | 0.44 | 0.23 |
| 18 | 0.14 | 0.13 |
| 19 | 0.33 | 0.14 |
| 20 | 0.30 | 0.17 |
| 21 | 0.82 | 0.86 |
| 22 | 0.41 | 0.57 |
| average | 0.4 | 0.9 |

| | | |
|---|---|---|
| * Individuals in whom a greater increase of CD3+/CD8+/GAD65 reactive T cells was observed after stimulation with the GAD65 peptide. | | |

At day 6, lymphocytes from 2 out of 22 normal controls included in the study showed a greater increase in the percentage of CD3+/CD8+ GAD65 reactive T cells after GAD65 peptide stimulation than in the basal conditions of IL-2 stimulation ( see Table 3, subjects indicated by asterisk*). Although this response apparently resembles that of patient lymphocytes, the peculiar pattern of 'low CD8' expression was not evident in Dot Plot (compare Fig 1 and Fig 2, panel E).

### b) Newly diagnosed T1D patients

In PBMCs of 22/22 newly diagnosed T1D, a low percentage of CD3+/CD8+ GAD65 reactive T cells was detected at day 6 from the beginning of culture after IL-2 stimulation (average 4.0%). The percentage of CD3+/CD8+/GAD65 reactive T cells after stimulation with IL-2 was variable in T1D patients, but was significantly higher than in healthy controls (4.0% vs. 0.4%, respectively, Table 4, Fig 2, Fig 4A and 4C).

**Table 4: Percentage of CD3/CD8 positive GAD65 reactive T cells detected in PBMC of newly diagnosed T1D patients both in basal conditions of IL-2 stimulation and under GAD65 peptide stimulation and analysed at day 6 from the beginning of the culture**

| T1D patient | IL-2 treated PBMC | GAD65 treated PBMC |
|---|---|---|
| | %CD3+/CD8+/GAD65 reactive T cells | %CD3+/CD8+/GAD65 reactive T cells |
| 1 | 1.4 | 73 |
| 2 | 0.32 | 17 |
| 3 | 0.1 | 12.5 |
| 4 | 2.44 | 14.08 |
| 5 | 0.2 | 1 |
| 6 | 0.2 | 1 |
| 7 | 5 | 19 |
| 8 | 20 | 98 |
| 9 | 8.6 | 85 |
| 10 | 0.75 | 68 |
| 11 | 14 | 22 |
| 12 | 11 | 70 |
| 13 | 0.6 | 82 |
| 14 | 0.2 | 21 |
| 15 * | 1.74 | 1.35 |
| 16 * | 2.45 | 2.64 |
| 17 | 0.21 | 0.81 |
| 18* | 2.47 | 3.19 |
| 19 | 15 | 58 |
| 20 | 0.28 | 2.59 |
| 21 | 0.28 | 2.28 |
| 22 | 1.31 | 6.39 |
| average | 4.0 | 30.0 |

| | | |
|---|---|---|
| * Individuals in whom no significant increase of CD3+/CD8+/GAD65 reactive T cells after stimulation with the GAD65 peptide was observed | | |

At day 6, there was a greater increase in CD3+/CD8+/ GAD65 reactive T cells in PBMC from T1D patients stimulated with GAD65 peptide than in those stimulated with IL-2 (30.0% vs 4.0%, Fig 2, Fig 4A-B and 4C-D, Table 4).
Lymphocytes of 3 out of the 22 T1D patients included in the study did not show a significant increase at day 6 from the beginning of the culture in the percentage of CD3+/CD8+/ GAD65 reactive T cells after GAD65 peptide stimulation, apparently behaving like lymphocytes of normal controls (Table 2). The authors were able to study the PBMCs of one of these three patients (samples of the other two patients were no longer available) after a brief stimulation with the GAD65 peptide (2 days only instead of 4). The tetramer analysis was done at day 4, after the two subsequent days of IL-2 stimulation. A significant increase of the percentage of CD3+/CD8+/ GAD65 reactive T cells was seen after GAD65 peptide stimulation, compared to IL-2 stimulation (Fig 8), a phenomenon due to a more prompt activation response to the GAD65 peptide.

In conclusion, GAD65 autoreactive T cells can be detected in peripheral blood lymphocytes of newly diagnosed T1D patients by means of HLA class I tetramers.

### Characteristics of cells detected in T1D patients

CD8+ GAD65 reactive T cells detected in T1D patients after stimulation with the GAD65 peptide show on Dot Plot a peculiar pattern of low CD8 expression that is not seen in the normal control population after stimulation with the GAD65 peptide. This phenomenon can be interpreted as a consequence of a specific activation of the T lymphocyte population in T1D patients (Fig 2E; Fig 5D and Fig 7).

### Evaluation of T cell activation

The population of GAD65 reactive T cells was found to express the activation marker CD69, in both the normal and T1D population (Table 5 and Table 6, respectively).

**Table 5: Percentage of CD69 positive T cells in the CD3+/CD8+ cell population of healthy normal controls**

| Normal controls | IL-2 treated PBMC | GAD65 peptide treated PBMC |
|---|---|---|
| | % CD69+/CD3+/CD8+ T cells | % CD69+/CD3+/CD8+ T cells |
| 1 | 1.3 | 1.7 |
| 2 | NT | NT |
| 3 | 1.2 | 2.1 |
| 4 | 0.4 | 0.75 |
| 5 | 11.32 | 13.51 |
| 6 | 19 | 17 |
| 7 | 29 | 26 |
| 8 | 12.3 | 13.4 |
| 9 | 22 | 18 |
| 10 | 22 | 19 |
| 11 | 26 | 18 |
| average | 14.4 | 12.9 |

**Table 6: Percentage of CD69 positive T cells in the CD3+/CD8+ T cell population of newly-diagnosed T1D patients.**

| T1D patients | IL-2 treated PBMC | GAD65 peptide treated PBMC |
|---|---|---|
| | % CD69/ CD3/CD8 positive T cells | % CD69/ CD3/CD8 positive T cells |
| 3 | 2 | 22 |
| 4 | 3.7 | 13.5 |
| 7 | 1.55 | 10.94 |
| 8 | 21 | 24 |
| 9 | 14 | 39 |
| 10 | 2 | 28 |
| 13 | 25 | 42 |
| 14 | 1.2 | 18 |
| 15 | 3.56 | 4.63 |
| 16 | 0.7 | 1.5 |
| 17 | 2.51 | 3.2 |
| 18 | 24 | 28 |
| 19 | 8 | 20 |
| 20 | 4.73 | 9.87 |
| average | 8.1 | 18.9 |

At day 6 from the beginning of the culture, no significant increase in CD69 positive T cells was detected in the healthy controls after treatment with the GAD65 peptide (12.9%) compared to IL-2 treatment (14.4%, Table 5). Conversely, in T1D patients, a significant increase in the CD69 positive cells was seen at day 6 from the beginning of the culture in the GAD65 reactive T cell population after stimulation with the GAD65 peptide (18.9%) compared to IL-2 (8.1%, Table 6).

### Specificity of the response to the GAD65 peptide

In order to evaluate the specificity of the response to the GAD65 peptide (evaluated by staining the stimulated population with the HLA-A*0201 GAD65 peptide tetramer), mononuclear cells of healthy control individuals and of T1D patients were cultured in parallel with IL-2, Flu and GAD65 peptide (Fig 5 and 6). In the T1D patients, at day 6 from the beginning of the culture, in the conditions described above, a significant increase in the population of CD3⁺/CD8⁺/GAD65 reactive T cells was evident after stimulation with the GAD65 peptide (6.39% of CD3+/CD8+/GAD65 reactive T cells), but not after stimulation with the Flu peptide (1.31% of CD3+/CD8+/GAD65 reactive T cells vs 1.89% after stimulation with IL-2, Fig. 5B,D,F). In the normal controls, no significant increase of the population of CD³+/CD⁸+/GAD65 reactive T cells was observed after stimulation with the GAD65 peptide, nor with the Flu peptide (Figure 6B, D,F).

### Evaluation of activation-induced apoptosis

The authors investigated the presence of apoptosis in lymphocytes of a control healthy individual and a T1D patient at day 4 or day 6 from the beginning of the culture after stimulation with the GAD65 peptide. Figure 9 shows the percentage of CD8+/GAD65 reactive lymphocytes in early apoptosis. In lymphocytes of a healthy control donor evaluated at day 4, the percentage of CD8+/GAD65 reactive lymphocytes was very low, and for this reason the evaluation of Annexin V positive cells was not relevant (Fig 9 B-C).

At day 4 from the beginning of the culture with the GAD65 peptide, a percentage of CD8+/GAD65 reactive lymphocytes shows signs of apoptosis, a phenomenon that can be due to the typical process of activation of CD8+ lymphocytes (Fig 9, panels E-F).

At day 6 of GAD65 peptide treatment (Fig 10), in the same samples examined, the population of Annexin V positive cells was no longer detectable, probably due to the rupture and disappearance of the antigen-activated CD8+ cells.

These data demonstrate the presence of early apoptotic phenomena at day 4 in lymphocytes of the considered patient that are a consequence of the early activation of a subset of CD8+/GAD65 reactive T cells after antigenic exposure.

### REFERENCES

1. Fierabracci A, Bottazzo GF, Springer Semin Immunopathol 24, 243-259 (2002)
2. Peakman M et al, Diabetes 50, 1749-1754 (2001)
3.Kotzin et al. PNAS 97, 291-296 (2000)
4. Reijonen H et al., Diabetes 51, 1375-1382 (2002)
5.Reijonen H et al., Ann. NY Acad. Sci. 1005, 82-87 (2003) Review
6. Gebe JA et al, Eur J Immunol 34, 3337-3345 (2004)
7. Fourneau J-M et al, J Immunol Methods 285, 253-264 (2004)
8.Ogg GS et al., J. Exp. Med. 188, 1203-1208 (1998)
9.Barber LD et al., Transplantation 77, 1604-1609 (2004)
10.Panina-Bordignon P et al., J. Exp. Med. 181, 1923-1927 (1995)
11. Strausberg RL et al, PNAS 99, 16899-16903 (2002)

## Claims

1. An *in vitro* method for detecting glutamic acid decarboxylase, isoform 65 (GAD65) specific T cells in a biological sample that consists of the following steps:
a) isolating mononuclear cells (PBMCs) from peripheral blood obtained from a subject;
b) stimulating said isolated PBMCs with an appropriate amount of the GAD65 peptide having the sequence VMNILLQYV (SEQ ID No. 1) added to the culture medium, in order to obtain autoantigen-specific stimulated PBMCs;
c) washing at least once said autoantigen-specific stimulated PBMCs;
d) stimulating said autoantigen-specific stimulated PBMCs with an appropriate amount of at least one cytokine to obtain fully stimulated PBMCs;
e) incubating in appropriate conditions said fully stimulated PBMCs with an appropriate amount of multimeric HLA class I labelled complexes, constructed with a peptide derived from GAD65 having the sequence VMNILLQYV (SEQ ID No. 1) and wherein the multimeric complexes are tetrameric or pentameric.
f) identifying and quantifying the percentage of T cells that are bound to said complexes and comparing said percentage to appropriate control values.

2. Method according to claim 1, in which the cytokine is IL-2.

3. Method according to claim 1, in which complexes are labelled with phycoerythrin.

4. An *in vitro* method for the prediction of Type 1 diabetes (T1D) onset, and/or to follow up the effects of therapeutics and in particular of specific immunotherapies in the pre-clinical period of the disease, essentially consisting of the steps of the method according to the previous claims.

5. Kit for performing the method according to claim 1 including:
a) one peptide derived from GAD65 having the sequence VMNILLQYV (SEQ ID No. 1);
b) at least one cytokine;
c) one multimeric labelled complex composed of HLA class I and the peptide derived from GAD65, wherein the multimeric complex is tetrameric or pentameric;
d) a first monoclonal antibody labelled with a first fluorochrome, directed against the human CD8 molecule; and
e) a second monoclonal antibody labelled with a second fluorochrome different from the first one, directed against the human CD3 molecule.

6. Kit according to claim 5, in which the cytokine is IL-2.

7. Kit according to claim 5, in which the complex is labelled with phycoerithrin

8. Kit according to claim 5, in which the first fluorochrome is Cy5 and the second fluorochrome is allophycocyanin (APC).

## Patentansprüche

1. In-vivo-Verfahren zum Feststellen von T-Zellen, die für Glutamatdecarboxylase, Isoform 65 (GAD65) spezifisch sind, in einer biologischen Probe, das aus folgenden Schritten besteht:
a) Isolieren mononukleärer Zellen (PBMC) aus dem peripheren Blut, das von einem Individuum erhalten wurde;
b) Stimulieren der isolierten PBMC mit einer dem Kulturmedium zugegebenen, geeigneten Menge des GAD65-Peptids, das die Sequenz VMNILLQYV (SEQ ID Nr. 1) aufweist, um autoantigenspezifische stimulierte PBMC zu erhalten;
c) Mindestens einmaliges Waschen der autoantigenspezifischen stimulierten PBMC;
d) Stimulieren der autoantigenspezifischen stimulierten PBMC mit einer geeigneten Menge mindestens eines Zytokins, um vollständig stimulierte PBMC zu erhalten;
e) Inkubieren der vollständig stimulierten PBMC mit einer geeigneten Menge multimerer HLA-Klasse-I-markierter Komplexe, die mit einem von GAD65 abstammenden Peptid konstruiert sind, das die Sequenz VMNILLQYV (SEQ ID Nr. 1) aufweist, unter geeigneten Bedingungen, wobei die multimeren Komplexe tetramer oder pentamer sind;
f) Identifizieren und Quantifizieren des Prozentanteils von T-Zellen, die an die Komplexe gebunden sind, und Vergleichen des Prozentanteils mit entsprechenden Kontrollwerten.

2. Verfahren nach Anspruch 1, wobei es sich bei dem Zytokin um IL-2 handelt.

3. Verfahren nach Anspruch 1, wobei die Komplexe mit Phycoerythrin markiert sind.

4. In-vitro-Verfahren zum Vorhersagen des Beginns von Typ-1-Diabetes (T1D) und/oder zur Nachbeobachtung der Auswirkungen von Therapeutika und insbesondere spezifischer Immuntherapien in der vorklinischen Phase der Krankheit, welches im Wesentlichen aus den Schritten des Verfahrens nach den vorherigen Ansprüchen besteht.

5. Kit zum Durchführen des Verfahrens nach Anspruch 1, einschließlich:
a) eines von GAD65 abstammenden Peptids, das die Sequenz VMNILLQYV (SEQ ID Nr. 1) aufweist;
b) mindestens eines Zytokins;
c) eines multimeren markierten Komplexes, das aus HLA-Klasse-I und dem von GAD65 abstammenden Peptid zusammengesetzt ist, wobei der multimere Komplex tetramer oder pentamer ist;
d) eines ersten monoklonalen Antikörpers, der mit einem ersten Fluorochrom markiert ist und gegen das menschliche CD8-Molekül gerichtet ist; und
e) eines zweiten monoklonalen Antikörpers, der mit einem zweiten Fluorochrom markiert ist und sich von dem ersten, gegen das menschliche CD3-Molekül gerichteten monoklonalen Antikörper unterscheidet.

6. Kit nach Anspruch 5, wobei es sich bei dem Zytokin um IL-2 handelt.

7. Kit nach Anspruch 5, wobei der Komplex mit Phycoerythrin markiert ist.

8. Kit nach Anspruch 5, wobei es sich bei dem ersten Fluorochrom um Cy5 und bei dem zweiten Fluorochrom um Allophycocyanin (APC) handelt.

## Revendications

1. Procédé *in vitro* de détection de cellules T spécifiques de l'acide glutamique décarboxylase, isoforme 65 (GAD65) dans un échantillon biologique, qui comprend les étapes suivantes :
a) l'isolement de cellules mononucléaires (PBMC) du sang périphérique prélevé sur un sujet ;
b) la stimulation desdites PBMC isolées avec une quantité adaptée du peptide GAD65, ayant la séquence VMNILLQYV (SEQ ID NO : 1), ajouté au milieu de culture, de manière à obtenir des PBMC stimulées spécifiques d'un autoantigène ;
c) le lavage au moins une fois desdites PBMC stimulées spécifiques d'un autoantigène ;
d) la stimulation desdites PBMC stimulées spécifiques d'un autoantigène avec une quantité adaptée d'au moins une cytokine pour obtenir des PBMC complètement stimulées ;
e) l'incubation dans des conditions adaptées desdites PBMC complètement stimulées avec une quantité adaptée de complexes multimériques marqués HLA de classe I, construits avec un peptide dérivé de GAD65 ayant la séquence VMNILLQYV (SEQ ID NO : 1), les complexes multimériques étant tétramériques ou pentamériques ;
f) l'identification et la quantification du pourcentage de cellules T qui sont liées auxdits complexes et la comparaison dudit pourcentage à des valeurs témoins adaptées.

2. Procédé selon la revendication 1, dans lequel la cytokine est l'IL-2.

3. Procédé selon la revendication 1, dans lequel les complexes sont marqués à la phycoérythrine.

4. Procédé *in vitro* de prédiction de l'apparition du diabète de type 1 (T1D), et/ou de suivi des effets des traitements et en particulier des immunothérapies spécifiques dans la période préclinique de la maladie, essentiellement constitué par les étapes du procédé selon les revendications précédentes.

5. Kit de réalisation du procédé selon la revendication 1, qui comprend :
a) un peptide dérivé de GAD65 ayant la séquence VMNILLQYV (SEQ ID NO : 1) ;
b) au moins une cytokine ;
c) un complexe multimérique marqué composé de HLA de classe I et du peptide dérivé de GAD 65, le complexe multimérique étant tétramérique ou pentamérique ;
d) un premier anticorps monoclonal marqué avec un premier fluorochrome, dirigé contre la molécule CD8 humaine ; et
e) un deuxième anticorps monoclonal marqué avec un deuxième fluorochrome différent du premier, dirigé contre la molécule CD3 humaine.

6. Kit selon la revendication 5, dans lequel la cytokine est l'IL-2.

7. Kit selon la revendication 5, dans lequel le complexe est marqué à la phycoérythrine.

8. Kit selon la revendication 5, dans lequel le premier fluorochrome est Cy5 et le deuxième fluorochrome est l'allophycocyanine (APC).


### SEQUENCE LISTING

<110> Fierabracci, Alessandra
   Bottazzo, Gian Franco
<120> Method for detecting GAD65 autoreactive T cells in newly diagnosed type I diabetic patients and in the prediabetic period
<130> BE 94744
<150> RM2005A000398
   <151> 2005-07-26
<160> 32
<170> PatentIn version 3.3
<210> 1
   <211> 9
   <212> PRT
   <213> Homo sapiens
<400> 1
   Val Met Asn Ile Leu Leu Gln Tyr Val
   1 5
<210> 2
<400> 2
   000
<210> 3
   <211> 9
   <212> PRT
   <213> Influenza virus
<400> 3
   Gly Ile Leu Gly Phe Val Phe Thr Leu
   1 5
<210> 4
   <211> 9
   <212> PRT
   <213> Homo sapiens
<400> 4
   Leu Leu Gln Glu Tyr Asn Trp Glu Leu
   1 5
<210> 5
   <211> 9
   <212> PRT
   <213> Homo sapiens
<400> 5
   Phe Leu Ile Glu Glu Ile Glu Arg Leu
   1 5
<210> 6
   <211> 9
   <212> PRT
   <213> Homo sapiens
<400> 6
   Phe Leu Gln Asp Val Met Asn Ile Leu
   1 5
<210> 7
   <211> 9
   <212> PRT
   <213> Homo sapiens
<400> 7
   Ile Leu Met His Cys Gln Thr Thr Leu
   1               5
<210> 8
   <211> 9
   <212> PRT
   <213> Homo sapiens
<400> 8
   Trp Leu Met Trp Arg Ala Lys Gly Thr
   1               5
<210> 9
   <211> 9
   <212> PRT
   <213> Homo sapiens
<400> 9
   Ala Leu Pro Arg Leu Ile Ala Phe Thr
   1               5
<210> 10
   <211> 9
   <212> PRT
   <213> Homo sapiens
<400> 10
   Gln Leu Ser Thr Gly Leu Asp Met Val
   1               5
<210> 11
   <211> 9
   <212> PRT
   <213> Homo sapiens
<400> 11
   Ala Met Met Ile Ala Arg Phe Lys Met
   1               5
<210> 12
   <211> 9
   <212> PRT
   <213> Homo sapiens
<400> 12
   Arg Met Met Glu Tyr Gly Thr Thr Met
   1 5
<210> 13
   <211> 9
   <212> PRT
   <213> Homo sapiens
<400> 13
   Phe Val Leu Leu Glu Tyr Val Thr Leu
   1 5
<210> 14
   <211> 9
   <212> PRT
   <213> Homo sapiens
<400> 14
   Lys Met Met Gly Val Pro Leu Gln Cys
   1 5
<210> 15
   <211> 9
   <212> PRT
   <213> Homo sapiens
<400> 15
   Arg Met Phe Pro Glu Val Lys Glu Lys
   1 5
<210> 16
   <211> 9
   <212> PRT
   <213> Homo sapiens
<400> 16
   Met Asn Ile Leu Leu Gln Tyr Val Val
   1 5
<210> 17
   <211> 9
   <212> PRT
   <213> Homo sapiens
<400> 17
   Leu Leu Gln Tyr Val Val Lys Ser Phe
   1               5
<210> 18
   <211> 9
   <212> PRT
   <213> Homo sapiens
<400> 18
   Glu Ile Leu Thr His Cys Gln Thr Thr
   1               5
<210> 19
   <211> 9
   <212> PRT
   <213> Homo sapiens
<400> 19
   Arg Leu Ile Ala Phe Thr Ser Glu His
   1               5
<210> 20
   <211> 9
   <212> PRT
   <213> Homo sapiens
<400> 20
   Ala Ala Thr Gly Lys Val Ala Cys Thr
   1               5
<210> 21
   <211> 9
   <212> PRT
   <213> Homo sapiens
<400> 21
   Lys His Tyr Asp Leu Ser Tyr Asp Thr
   1               5
<210> 22
   <211> 10
   <212> PRT
   <213> Homo sapiens
<400> 22
   Arg Met Met Glu Tyr Gly Thr Thr Met Val
   1 5 10
<210> 23
   <211> 10
   <212> PRT
   <213> Homo sapiens
<400> 23
   Asn Met Phe Thr Tyr Glu Ile Ala Pro Val
   1 5 10
<210> 24
   <211> 10
   <212> PRT
   <213> Homo sapiens
<400> 24
   Phe Leu Gln Asp Val Met Asn Ile Leu Leu
   1 5 10
<210> 25
   <211> 10
   <212> PRT
   <213> Homo sapiens
<400> 25
   Phe Thr Tyr Glu Ile Ala Pro Val Phe Val
   1 5 10
<210> 26
   <211> 10
   <212> PRT
   <213> Homo sapiens
<400> 26
   Leu Met Ser Arg Lys His Lys Trp Lys Leu
   1 5 10
<210> 27
   <211> 10
   <212> PRT
   <213> Homo sapiens
<400> 27
   Ala Leu Gln Cys Gly Arg His Val Asp Val
   1 5 10
<210> 28
   <211> 10
   <212> PRT
   <213> Homo sapiens
<400> 28
   Phe Leu His Ala Thr Asp Leu Leu Pro Ala
   1 5 10
<210> 29
   <211> 10
   <212> PRT
   <213> Homo sapiens
<400> 29
   Glu Leu Leu Gln Glu Tyr Asn Trp Glu Leu
   1 5 10
<210> 30
   <211> 10
   <212> PRT
   <213> Homo sapiens
<400> 30
   Phe Thr Ser Glu His Ser His Phe Ser Leu
   1 5 10
<210> 31
   <211> 10
   <212> PRT
   <213> Homo sapiens
<400> 31
   Asn Gln Leu Ser Thr Gly Leu Asp Met Val
   1 5 10
<210> 32
   <211> 10
   <212> PRT
   <213> Homo sapiens
<400> 32
   Leu Leu Gln Glu Tyr Asn Trp Glu Leu Ala
   1 5 10